# EUROPEAN PATENT SPECIFICATION

(11) **EP 3 497 598 B1**
(45) Date of publication and mention of the grant of the patent: **27.05.2020**
(21) Application number: 17748514.1
(22) Date of filing: 09.08.2017
(51) Int. Cl.: A61M 5/00, G16H 10/65, G16H 20/13

(54) **SAFETY APPARATUS AND METHOD TO TRACE, CONTROL AND MONITOR THE ADMINISTRATION OF MEDICAL SUBSTANCES**
SICHERHEITSAPPARAT UND METHODE ZUR VERFOLGUNG, KONTROLLE UND ÜBERWACHUNG DER ABGABE VON MEDIZINISCHEN SUBSTANZEN
APPAREIL DE SÉCURITÉ ET MÉTHODE POUR LA PERSÉCUTION, LE CONTRÔL ET LA SURVEILLANCE DE L'ADMINISTRATION DES AGENTS MEDICAUX

(30) Priority: 09.08.2016 IT 201600083934
(43) Date of publication of application: 19.06.2019
(73) Proprietor: Murata ID Solutions S.r.l., 43044 Collecchio (PR) (IT)
(72) Inventor: FANTONI GUERCI, Francesco, 43121 Parma (IT)
(74) Representative: Petraz, Gilberto Luigi
(86) International application number: PCT/EP2017/070214
(87) International publication number: WO 2018/029261

(56) References cited:
- WO-A2-2007/081947
- US-A1- 2009 043 253
- US-A1- 2009 259 336
- US-A1- 2012 305 428
- US-A1- 2014 155 827

## Description

### FIELD OF THE INVENTION

The present invention concerns a safety apparatus and method to trace, control and monitor the administration of medical substances, particularly but not exclusively, of blood liquids, biological liquids, pharmaceutical substances, vaccines or similar substances which are to be administered to a patient.

The present invention provides an identification association at least between a patient and the containers of medical substances to be administered to the patient.

### BACKGROUND OF THE INVENTION

Currently in the field of transfusion of blood components it is known to provide a first step in which the immunological compatibility of the patient with the blood components he is to receive is verified. During this step, after having taken and analyzed a sample of biological liquid, for example blood liquid, of the patient in the ward, a certain number of sacs of blood are assigned to the Transfusion Service (TS), and are made compatible on the basis of the blood sample arriving from the ward itself. In this way, for every patient who presumably needs transfusions, a certain number of sacs of blood components are "booked", ready to be transfused. Subsequently, there is a second step that is activated when the need for transfusing the blood components actually arises. In this case, a second blood sample is taken from the patient in the ward to perform a second check to confirm the identity of the patient for a safe transfusion.

When this check has been passed too, the compatible units are sent to the ward and transfused.

In particular, currently, while with serological examinations there is a particularly high inherent intrinsic safety of the blood, the safety of the transfusion is still quite deficient. During sample collection in the ward, during the association of the sample and the sac at the Transfusion Service (TS), or during administration to the patient of the contents of the sacs in the ward, errors can occur that could prejudice the patient's safety.

The highest error rate is primarily in identifying the patient when taking a blood sample to be sent for pre-transfusion control, as well as in the administration in the ward of the blood components issued by the Transfusion Service (TS).

The main negative consequence is a possible and significant risk of administering the wrong blood to the wrong patient.

Document US-A-2012/0305428 describes a medical safety apparatus for medical substances, such as drugs. The apparatus comprises a containing body in which the medical substance is stored, a closing lid for the containing body, a lock able to be selectively activated/deactivated to prevent or allow the lid to be opened, and an RFID reading device installed in the containing body to command the opening of the lock.

The apparatus includes an RFID identification element that can be associated with a patient. When the containing body is required to be opened, the RFID identification element of the patient is read by the RFID reading device, which if it detects a correct signal, sends a signal to open the lock.

Document US-A-2009/0259336 describes an administration device to administer tablets comprising a containing body for the tablets, a lid to close the containing body, a lock to close the lid of the containing body.

The lock is provided with an RFID reader that determines the opening of the lock when a correct RFID identification element is read which is associated with a user.

Document US-A-2009/0043253 describes a container, or box, to accommodate medical contents. The container comprises a lock provided with an RFID identification element which allows/prevents access inside the container. Access to the container is controlled through the interaction between an RFID reader and the RFID identification element. However, the RFID reader is not integrated into the container and therefore it is not possible to allow access to the contents of the container to a user who does not have an RFID reader. On the contrary, the presence of an RFID reader integrated with the container allows to verify and authorize the opening of the container in any environment without a remote RFID reader and disconnected from the container.

The solutions proposed in documents US-A-2012/0305428, US-A-2009/0259336 and US-A-2009/0043253, however, do not allow to control the medical substance that is to be administered to the patient. In fact, one of the problems that occur is the incorrect insertion of the medical substance into the containing body due to an error by the operators.

This problem is even more pronounced in the case of single-dosage transfusion sacs or medicines.

Document US-A-2014/0155827 describes a device that contains an emergency medicine and also comprises a sensor, an RFID identification element and a memory. The sensor can be a temperature sensor that continuously measures the temperature of the medicine and can send the temperature values detected to a processor, to compare them with approved temperature values.

Document WO-A-2007/081947 describes a device for storing and distributing a medicine, comprising a sensor to perceive the state of preservation of the medicine by detecting the humidity and heat, or if the medicine has expired.

One purpose of the present invention is to obtain an apparatus and to perfect a method to control the administration of a medical substance that eliminates the risk of administering the latter to the wrong patient.

Another purpose of the present invention is to increase the safety of the administration of the medical substance, particularly in the case of transfusion of blood components during sample taking in the ward, during the association of the sample and the sac at the Transfusion Service (TS), during the movement of the sacs, and/or when administering the sacs in the ward.

The Applicant has devised, tested and embodied the present invention to overcome the shortcomings of the state of the art and to obtain these and other purposes and advantages.

### SUMMARY OF THE INVENTION

The present invention is set forth and characterized in the independent claims, while the dependent claims describe other characteristics of the invention or variants to the main inventive idea.

In accordance with the above purposes, the present invention concerns a safety apparatus to trace, control and monitor the administration of medical substances, which comprises:
- a container of a medical substance;
- a containing body with a compartment in which to put the container, a lid to selectively close the compartment, a lock associated with the containing body and with the lid and able to be selectively activated/deactivated to prevent or allow the opening of the lid.

According to the present invention, the apparatus also comprises a first identification element associated directly with the container of the medical substance;
- a wearable element associable with a patient and provided with a second identification element;
- a reading device configured to read data of the first identification element and the second identification element, to compare said data, and to command the selective opening of the lock. The reading device is also solidly associated with the containing body and is connected to the lock.

The presence of an identification element integrated in the container of the medical substance allows to link the information recorded therein with the medical substance contained, following the movements carried out by the latter.

In this way, the errors that can be generated during the transport or administration of the medical substance can be considerably reduced, or possibly eliminated.

Moreover, the reading device integrated with the containing body allows to carry out a comparison between the data of the first identification element and of the second identification element at any time. This aspect would not be applicable if there were a reading device not directly associated with the containing body of the container of the medical substance.

According to the present invention, the medical substance can be chosen from a group comprising at least one of either a blood component, blood or its parts, or a saline solution, a drug, or other liquids which are usually infused into a patient via a drip, under medical control and which need a compatibility control with the sensitive data of the patient.

According to some embodiments, the wearable element can advantageously be a bracelet, typically already used in the hospital sector, a pendant, a brooch or other similar element that can be worn by a patient.

According to one embodiment, the apparatus can comprise two first identification elements, of which one first identification element is associated with the container while the other is associated with the containing body in order to carry out, advantageously, a double control by the reading device.

The solution described above allows to associate the container, by means of data contained in the first identification element, with a predetermined patient. The opening of the lid is conditioned by a comparison of the data associated with the first and the second identification elements. In fact it can be provided that the data associated with the container or the containing body contain at least information relating to the patient that is to receive the medical substance and that, when the container arrives on the ward, the reading device reads both the data of the first and of the second identification elements. Subsequently, the reading device, carrying out a comparison between the first and the second data, allows or prevents the opening of the lid.

The present invention thus eliminates the risk of administering the wrong medical substance to the wrong patient and increases the safety of the administration, particularly in the case of blood component transfusions.

There is, in fact, a secure identification of the patient when the administration is done on the ward.

Merely by way of example, it can be provided that when a blood sample is taken from a patient to then be sent for pre-transfusion control, information relating to that patient can be associated with the test tube containing the sample, for example the same data contained in the second identification element. The transfusion service, having received the test tube and carried out the necessary analyses, identifies the medical substances which can be administered to that patient. It therefore associates the first identification element with the container containing the medical substances, or with the containing body, also associating information relating to the patient for whom the medical substances are intended with the first identification element. The container is put in the containing body and the lid is closed, activating the corresponding lock. The latter can be deactivated, allowing the opening of the lid, only when the reading device reads corresponding information in the second identification element.

According to one embodiment, the containing body comprises a sensor to detect technical parameters, such as temperature, pressure, humidity of the compartment where the container is located, in order to constantly monitor the state of preservation of the medical substance present in the containing body.

In this way, advantageously, the integrity of the medical substance is constantly controlled during the transfers and transports from the Transfusion Service (TS) to the ward concerned, during its preservation in the destination structures and/or while "parked" in the operating theater.

If the technical parameters deviate from predefined threshold values, the lock can prevent the opening of the lid and error signals can be sent in this regard.

The present invention also concerns a safety method to trace, control and monitor the administration of medical substances, which comprises:
- introducing a medical substance into a container;
- introducing the container into a compartment of a containing body.

According to one aspect of the present invention, the method comprises:
- associating a first identification element directly with the container of the medical substance;
- closing the compartment with a lid;
- activating a lock, associated with the compartment and the lid to prevent the opening of the lid;
- associating a second identification element with a wearable element associable with a patient;
- reading and comparing data contained in the first identification element and the second identification element with a reading device solidly associated with the containing body and connected to the lock;
- sending a command to open the lock with the reading device as a function of the comparison.

### BRIEF DESCRIPTION OF THE DRAWINGS

These and other characteristics of the present invention will become apparent from the following description of some embodiments, given as a non-restrictive example with reference to the attached drawings wherein:
- fig. 1 is a perspective view of a safety apparatus to trace, control and monitor the administration of medical substances according to the present invention;
- fig. 2 is a front view of a containing body;
- fig. 3 is a schematic illustration of a safety apparatus to trace, control and monitor the administration of medical substances in accordance with a possible embodiment.

To facilitate comprehension, the same reference numbers have been used, where possible, to identify identical common elements in the drawings. It is understood that elements and characteristics of one embodiment can conveniently be incorporated into other embodiments without further clarifications.

### DETAILED DESCRIPTION OF SOME EMBODIMENTS

We shall now refer in detail to the various embodiments of the present invention, of which one or more examples are shown in the attached drawings. Each example is supplied by way of illustration of the invention and shall not be understood as a limitation thereof. For example, the characteristics shown or described insomuch as they are part of one embodiment can be adopted on, or in association with, other embodiments to produce another embodiment.

It is understood that the present invention shall include all such modifications and variants.

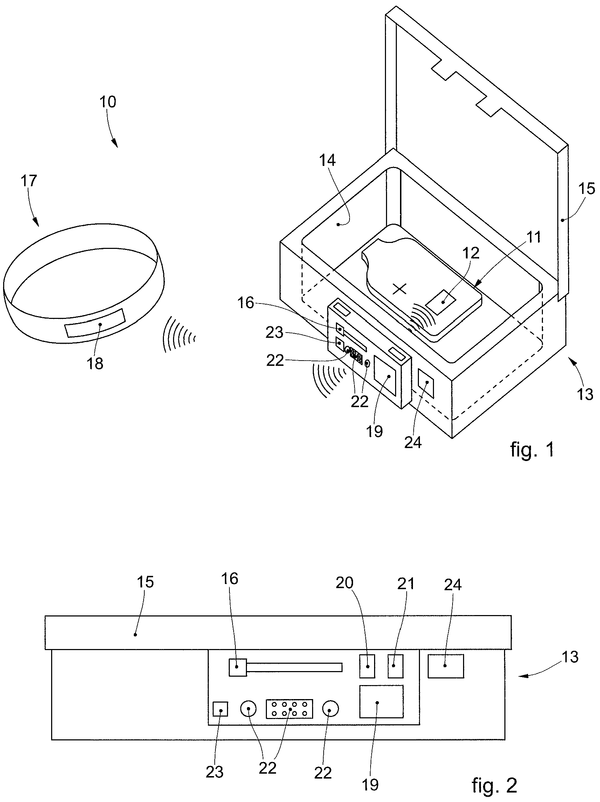

With reference to figs. 1 to 3, we shall now describe possible embodiments of the present invention relating to a safety apparatus 10 to trace, control and monitor the administration of medical substances.

Without this constituting a limitation to the present invention, by medical substances we mean biological liquids, pharmaceutical substances, vaccines.

The term biological liquid includes blood components, such as blood and blood derivatives, to be used, for example, in a transfusion process in a hospital-medical center provided with a Transfusion Service (TS) and areas dedicated to patient admissions.

Although hereafter we will refer to biological liquids, the present description can be understood to also apply in a substantially similar manner to medical substances in general.

The safety apparatus 10 comprises at least one container 11 of the medical substance, which can be, for example, a blood collection sac suitable to perform transfusions, or any container suitable to contain medical substances such as a test tube, blister, or suchlike.

The safety apparatus 10 also comprises a containing body 13 having a compartment 14 to put the container 11 inside it and a lid 15 to selectively close the compartment 14.

The containing body 13 is a separate and independent component from the container 11.

According to embodiments described here, the containing body 13 and/or lid 15 or at least a part thereof can be made of a polymer material, for example transparent, to make the contents visible to a user even with the lid 15 closed.

The containing body 13 and/or lid 15 can be made of materials that are easily sanitized with detergents and/or disinfectants.

Furthermore, in the event of damage to the container 11 or incorrect closure of the latter, the biological liquid remains confined to the containing body 13. The possible transparency of the containing body 13, or part of it, allows the operators to verify the possible dispersal of medical substance in the compartment 14 due to damage to the container 11.

The safety apparatus 10 comprises a lock 16 associated with the containing body 13 and the lid 15 and is able to be selectively activated/deactivated to prevent or allow the opening of the lid 15.

The lock 16 can be activated/deactivated electronically.

According to possible formulations of the present invention, the lock 16 can be of the normally closed type or one that keeps the lid 15 in a condition to close the compartment 14 until it is actuated to determine the opening of the lock 16 and therefore of the lid 15.

According to one aspect of the present invention, a first identification element 12 is associated with at least one of either the container 11 or the containing body 13.

The first identification element 12 can contain at least identification data of the patient for whom the medical substance contained in the container 11 is intended.

The first identification element 12 can also comprise data relating to the medical substance contained in the container 11.

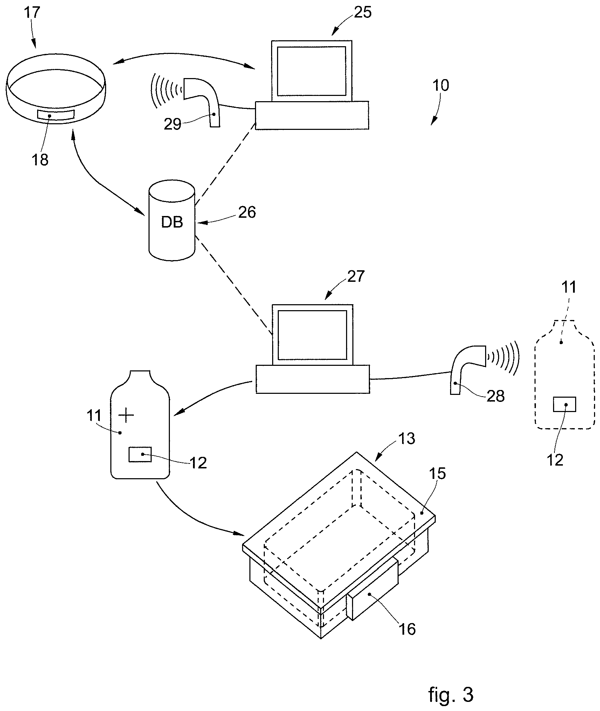

According to the solution shown in figs. 1 and 3, the first identification element 12 is directly associated with the container 11 of the medical substance.

According to a possible variant embodiment, not shown, the first identification element 12 is associated with the containing body 13, for example the lid 15 of the latter or its lock 16.

The safety apparatus 10 also comprises a wearable element 17 which can be associated with a patient and provided with a second identification element 18.

At least one patient identification datum, for example at least one of either the name, surname, tax code, nosographic code, or a unique identification number can be associated with the second identification element 18.

According to a possible solution, the wearable element 17 is a bracelet attachable to the wrist of a patient. According to variant embodiments, the wearable element 17 can also possibly comprise a cord, brooch, or necklace that can be worn by a patient.

According to a possible solution shown, for example, in figs. 1 and 3, the first identification element 12 and/or the second identification element 18 can comprise an RFID (Radio Frequency Identification) configured to memorize first data and respectively second data.

The first identification element 12 and/or the second identification element 18 can comprise a HF (High Frequency) RFID transponder or tag, for example functioning at a frequency of 13.56 MHz.

The first identification element 12 and/or the second identification element 18 of the RFID type can be provided with respective non-rewritable memories in which a univocal identification number, also called UID, is memorized.

In particular, at least the univocal identification number of the second identification element 18 can univocally identify the patient to whom the medical substance is to be administered. The univocal identification number can be assigned to the patient, for example, when the latter is registered in a hospital ward.

According to a variant embodiment, possibly combinable with the embodiments described here, the first identification element 12 and/or the second identification element 18 of the RFID type can comprise a rewritable memory in which data are memorized, for example relating to the patient who is to receive the substance, or data relating to the properties of the medical substance contained in the container 11.

According to a possible variant embodiment, not shown, the first identification element 12 and/or the second identification element 18 can comprise a graphical code, such as a bar code, a QR code, an identification number, or another two-dimensional code.

In this case too, the identification code of the first identification element 12 and the second identification element 18 can be associated with a specific patient.

According to another solution, at least the first identification element 12 can comprise a programmable memory in which data can be memorized, readable by electronic devices.

If the first identification element 12 is associated with the containing body 13, the latter can be provided with a reception device 23 configured to receive data and memorize it in the programmable memory of the first identification element 12.

The first identification element 12 and/or the second identification element 18 can be associated with an adhesive label which can be fixed to the container 11 and/or to the containing body 13 and respectively to the wearable element 17.

According to one aspect of the present invention, the apparatus 10 comprises a reading device 19 configured to read the data of the first identification element 12 and the second identification element 18, to compare said data and to command the selective opening of the lock 16.

In this way, when biological liquids are required to be administered to a patient, the supplier body receives the patient's identification data and the type of biological liquid required.

An operator of the body that supplies the biological liquids identifies the biological liquids compatible with the request received and selects one or more containers 11 that contain the desired biological liquid.

The operator associates the first identification element 12 with at least one of the container 11 and/or the containing body 13.

In particular, it can be provided that the operator writes data in the first identification element 12, for example in the RFID, or in the programmable memory, and these data can be directly read and compared to those associated with the second identification element 18 to determine the opening of the containing body 13.

By way of example only, it can be provided that the operator will write in the first identification element 12 the same data that are associated with the second identification element 18 and which, for example, have been transmitted to it together with the request for the medical substance.

According to a possible variant embodiment, it can be provided that the data of the first identification element 12 are memorized in a database, accessible for example by the reader device 19, and these data are associated with those of the patient who is to receive the medical substance.

According to an alternative, for example if the first identification element 12 is associated with the containing body 13, it can be provided that the container 11 is inserted and closed in the containing body 13 and that in the memory or in the RFID associated with the latter, data to be compared with those of the second identification element 18 are memorized.

According to a possible solution, the apparatus can comprise a reading/writing device 28 configured to read and/or write data from, or respectively into, the first identification element 12.

According to possible embodiments, the safety apparatus 10 can comprise a management unit 27 used by the body that supplies the biological liquids, in this case located at the Transfusion Service, which contains at least the data of the medical substance contained in the containers 11, data relating to the patients who will receive said medical substances, data relating to the association of a determinate medical substance with a determinate patient.

The management unit 27 can be connected to the reading/writing device 28 to manage the data input modes in the first identification element 12.

According to an advantageous solution, the reading device 19 is solidly associated with the containing body 13. In this way, the reading device 19 can directly read the data contained in the first identification element 12 and, by bringing the wearable element 17 closer thereto, also the data contained in the second identification element 18. This solution is particularly secure since it causes a direct comparison of the data without the interposition of possible processing or transfers of information.

In particular, according to this embodiment, it is provided that the reading device 19 is connected, directly, or wirelessly, to the lock 16 to command its opening.

According to another embodiment, the reading device 19 can be installed on a perimeter edge of the containing body 13, for example along the development of the edge defining the opening to access the compartment 14, to reliably detect the first identification element 12.

According to a possible alternative, the reading device 19 can be a separate component with respect to the containing body 13 and can be selectively associated with the latter on each occasion, and in particular with the lock 16 at the time when the opening of the containing body 13 is required. This allows to obtain extremely economical containing bodies 13 in which the electronic reading part is associated on each occasion with the containing body 13 only when the opening of the lock 16 is required.

According to this solution, and in the case where the first identification element 12 and the second identification element 18 are defined by a graphical code, it can be provided that the reader 19 reads the data contained therein, compares them, and if they have previously been associated with each other, determines the opening of the lock 16.

According to a possible formulation of the invention, the safety apparatus 10 comprises an auxiliary management unit 25, for example located in the patient admission area at the time of hospitalization and configured to memorize at least data relating to the patient, for example a reference number of the patient. In particular, the auxiliary management unit 25 can at least memorize information relating to the association of a determinate second identification element 18 with a determinate patient.

The auxiliary management unit 25 can be provided with a writing device 29 configured to electronically write the patient identification data in the second identification element 18.

This operation can be performed on the second identification element 18 both when the patient is admitted to the ward and also when the administration of biological liquids is required.

It is quite clear that the safety apparatus 10 can comprise a plurality of these auxiliary management units 25, located in different zones, for example, of one or more hospitals.

According to possible solutions, the auxiliary management unit 25 and the management unit 27 can be connected to each other, for example with a data transmission network, via cable or in wireless mode, to obtain a reciprocal exchange of information.

According to possible embodiments, at least one of either the auxiliary management unit 25 or the management unit 27 can be connected to a general database 26 to memorize and transfer health data relating to the patient.

According to one embodiment, the containing body 13 can comprise at least one sensor 20 configured to detect physical parameters useful for monitoring the state of preservation of the biological liquid in the container 11.

For example, the physical parameters can be temperature, pressure, density, or suchlike.

According to another embodiment, the containing body 13 can also comprise a memory 21 configured to receive and memorize the physical parameters detected by the sensor 20.

The data memorized by the memory 21 can be analyzed, for example by a control unit, before the biological liquids are administered to the patient, to verify whether the biological liquids are altered due to incorrect storage conditions, for example during transport.

According to a possible embodiment, the memory 21 can be connected to the lock 16 and, if the data detected by the sensor 20 deviate from threshold values, it can be configured to send a blocking signal to stop the opening of the lock 16. The blocking signal can also be associated with an acoustic and/or visual indicator.

According to another embodiment, the containing body 13 can comprise at least one indicator 22 to communicate the state of activation/deactivation of the lock 16.

According to the embodiments described here, the indicator 22 can be a luminous indicator, for example a colored light able to emit a different color depending on the activation or deactivation of the lock 16.

According to embodiments described here, the indicator 22 is the acoustic type able to differentiate the sound emitted according to the activation or deactivation of the lock 16.

According to one embodiment, the containing body 13 can comprise a transmitter 24 configured to communicate with electronic devices such as smartphones, tablets, PCs, or suchlike.

The transmitter 24 can communicate with a Bluetooth®, Energy® or ZigBee module, or suchlike.

The transmitter 24 can be configured to transmit information relating, for example, to at least one of the data memorized in the first identification element 12, the physical preservation parameters of the biological liquid during transport, and possible openings of the containing body 13 that occurred before administration to the patient.

According to possible solutions, the lock 16 can also comprise a timer provided to automatically close the lock 16 if, after a predetermined time interval has passed since the command to open the lock 16, the lid 15 has not been opened.

The present invention also concerns a safety method to trace, control and monitor the administration of medical substances.

We shall now describe a possible formulation of the safety method.

At the moment when there is a need to administer biological liquids, for example blood components, to the patient, a predetermined safety method is initiated.

Firstly, a first sample is taken from the patient for pre-transfusion tests.

The first sample is transferred to a first test tube with which the patient's data is associated, for example the same data contained in the second identification element 18.

According to possible solutions, it can be provided that an identification label is associated with the first test tube, and the name or identification number of the patient is associated with said label.

According to a variant, a third identification element, for example an RFID, can be associated with the first test tube, in which the data of the patient is memorized.

Subsequently, the first test tube containing the blood samples is sent to the Transfusion Service (TS).

The Transfusion Service (TS) uploads the patient's data, verifies they are correct, and performs the blood tests as prescribed by current legislation.

In particular, it is provided to carry out typing tests that determine the blood cell antigens.

Based on the results of the analysis of the blood samples, a number of containers 11 are identified, whose biological liquid is compatible with the patient.

Each container 11 identified is temporarily assigned to the patient through the management unit 27.

When the need for transfusion of the blood components arises, a second blood sample is taken from the patient, introduced into a second test tube with which a label or third identification element can be associated, in a manner substantially analogous to the one described above for the first test tube.

The second test tube is transferred to the Transfusion Service where another typing control is carried out, verifying that the information of the results of the typing analyses performed on the second sample correspond with those of the first sample.

At this point, if the information corresponds, the previously identified container 11 is definitively assigned to the patient, memorizing the information on the patient in the first identification element 12, for example by means of the reading/writing device 28.

Once the writing operation has been performed, the first identification element 12 will have memorized inside it both its information, at least its univocal identification element, and also the patient's data, for example the same data present in the second identification element 18.

As an alternative to memorization, it can be provided that the first identification element 12 is printed, for example, with a graphical identification code.

In this condition, there can be at least two solutions, possibly even combinable:
- the first identification element 12 is associated with the container 11, and the latter is subsequently inserted and closed in the containing body 13;
- the first identification element 12 is associated with the containing body 13.

The container 11 is inserted and closed in the containing body 13.

When the container 11 is inserted or the containing body 13 is closed, it can be provided that the reading device 19, in this case solid with the containing body 13, reads the information contained in the first identification element 12 and allows to activate the lock 16 and therefore to selectively close the lid 15.

The container 11 is ready to be delivered to the requesting ward.

The method also provides a cross-checking between the information in the first identification element 12 and the second identification element 18.

In particular, once the containing body 13 with the container 11 inside it arrives in the ward, it can be provided that when the biological liquids are administered, the wearable element 17 is brought closer to the reading device 19 so that the latter reads the data contained in the second identification element 18 and compares them with the data contained in the first identification element 12.

In the event that there is no correspondence between the data contained in the first identification element 12 and those of the second identification element 18, the release of the lock 16 will be prevented, and therefore the extraction of the container 11 as well.

Otherwise, it will be possible to extract the container 11 from the containing body 13 to perform the administration to the patient.

Throughout the period when the container 11 is contained in the containing body 13, each single opening and closing action of the containing body 13 is memorized in the memory 21.

From the information contained in the database 26 it is possible to know when and which patient received a certain container 11 of blood components, by whom it was administered, and it is therefore possible to define a historical archive of the transfusions performed for each patient.

Furthermore, the safety apparatus 10 allows to memorize a spatial tracking of the path followed by the container 11 from the Transfusion Service (TS) until it is administered to the patient.

For example, it can be provided that the safety apparatus 10 comprises a plurality of detection units located in different passage zones of the containing body 13 at the exit of the transfusion center and/or at the entrance to the wards.

The detection units can be configured to identify the passage of that particular container 11, for example by reading the data contained in the first identification element 12.

A software application allows to manage the safety device 10 and more generally the transfer of information.

It is clear that modifications and/or additions of parts may be made to the safety apparatus 10 and method as described heretofore, without departing from the field and scope of the present invention.

It is not excluded that possible solutions provide that in association with the lock 16 with electronic opening there is also the possibility of mechanical activation/deactivation, for example by means of a key, or a button, or suchlike, to be used in emergency conditions in the event that the electronic opening was damaged.

According to possible variant embodiments, it can be provided that the containing body 13 is provided with facilitated opening lines, configured to allow an emergency opening of the containing body 13, thereby preventing any damage to the container 11 contained in the compartment 14. If the containing body 13 is opened along the facilitated opening lines, there will be damage to the containing body 13.

In other embodiments it can also be provided that the reading device 19, solidly associated with the containing body 13, is configured to read the data relating to an operator who initiates the method to open the containing body 13. For example, it can be provided that the reading device 19 is configured to read the data for example of a badge assigned to the operator and that said data are memorized, for example, in the memory 21 of the lock 16.

It is also clear that, although the present invention has been described with reference to some specific examples, a person of skill in the art shall certainly be able to achieve many other equivalent forms of safety apparatus 10 and method, having the characteristics as set forth in the claims and hence all coming within the field of protection defined thereby.

## Claims

1. Safety apparatus to trace, control and monitor the administration of medical substances, comprising:
- a container (11) of a medical substance;
- a containing body (13) with a compartment (14) in which to put said container (11), a lid (15) to selectively close said compartment (14), a lock (16) associated with said containing body (13) and with said lid (15) and able to be selectively activated/deactivated to prevent or allow the opening of said lid (15);
**characterized in that** it comprises:
- a first identification element (12) associated directly with said container (11) of the medical substance;
- a wearable element (17) associable with a patient and provided with a second identification element (18);
- a reading device (19) configured to read data of said first identification element (12) and said second identification element (18), to compare said data, and to command the selective opening of said lock (16), said reading device (19) being solidly associated with said containing body (13) and being connected to said lock (16).

2. Apparatus as in claim 1, **characterized in that** at least one of either said first identification element (12) or said second identification element (18) comprises an RFID configured to memorize said data.

3. Apparatus as in claim 1 or 2, **characterized in that** it comprises a reading/writing device (28) configured to read and/or write said data from the, or respectively in, the first identification element (12).

4. Apparatus as in any claim hereinbefore, **characterized in that** said containing body (13) comprises at least a sensor (20) configured to detect physical parameters for monitoring the state of conservation of said medical substance in said container (11).

5. Safety apparatus as in claim 4, **characterized in that** said containing body (13) comprises a memory (21) configured to receive and memorize the physical parameters detected by said sensor (20).

6. Safety apparatus as in any claim hereinbefore, **characterized in that** it comprises two first identification elements (12), of which one of the first identification elements (12) is associated with the container (11) and the other is associated with the containing body (13).

7. Safety method to trace, control and monitor the administration of medical substances, comprising:
- introducing a medical substance into a container (11);
- introducing said container (11) into a compartment (14) of a containing body (13);
**characterized in that** it comprises:
- associating a first identification element (12) directly with said container (11) of the medical substance;
- closing said compartment (14) with a lid (15);
- activating a lock (16), associated with said compartment (14) and said lid (15) to prevent the opening of said lid (15);
- associating a second identification element (18) with a wearable element (17) associable with a patient;
- reading and comparing data contained in said first identification element (12) and said second identification element (18) with a reading device (19) solidly associated with said containing body (13) and being connected to said lock (16);
- sending a command to open said lock (16) with said reading device (19) as a function of said comparison.

8. Method as in claim 7, **characterized in that** at least one of either said first identification element (12) or said second identification element (18) comprises an RFID configured to memorize said data, **and in that** it comprises at least a memorization of data in said first identification element (12) and/or said second identification element (18).

9. Method as in claim 7 or 8, **characterized in that** it comprises detecting and controlling physical parameters of said container (11) by means of at least a sensor (20), and recorded by a memory (21).

10. Method as in claim 9, **characterized in that** it comprises memorizing said physical parameters in said memory (21), and analyzing said physical parameters memorized with threshold values.

## Patentansprüche

1. Sicherheitsvorrichtung zum Verfolgen, Steuern und Überwachen der Verabreichung von medizinischen Substanzen, welche umfasst:
- einen Behälter (11) einer medizinischen Substanz;
- einen Aufbewahrungskörper (13) mit einer Kammer (14), in welche der Behälter (11) eingebracht werden kann, einen Deckel (15) zum wahlweisen Schließen der Kammer (14), und einem Schloss (16), welches dem Aufbewahrungskörper (13) und dem Deckel (15) zugeordnet ist und wahlweise aktiviert/deaktiviert werden kann, um das Öffnen des Deckels (15) zu verhindern oder zu erlauben;
wobei die Sicherheitsvorrichtung **dadurch gekennzeichnet ist, dass** sie umfasst:
- ein erstes Identifizierungselement (12), welches dem Behälter (11) der medizinischen Substanz direkt zugeordnet ist;
- ein tragbares Element (17), welches einem Patienten zugeordnet werden kann und mit einem zweiten Identifizierungselement (18) ausgestattet ist;
- ein Auslesevorrichtung (19), welche eingerichtet ist, Daten des ersten Identifizierungselements (12) und des zweiten Identifizierungselements (18) auszulesen, die Daten zu vergleichen und das wahlweise Öffnen des Schlosses (16) anzuordnen, wobei die Auslesevorrichtung (19) fest dem Aufbewahrungskörper (13) zugeordnet ist und mit dem Schloss (16) verbunden ist.

2. Vorrichtung nach Anspruch 1, welche **dadurch gekennzeichnet ist, dass** mindestens eines aus dem ersten Identifizierungselement (12) oder dem zweiten Identifizierungselement (18) einen RFID umfasst, welcher eingerichtet ist, die Daten zu speichern.

3. Vorrichtung nach Anspruch 1 oder 2, welche **dadurch gekennzeichnet ist, dass** sie eine Auslese-/Schreibvorrichtung (28) umfasst, welche eingerichtet ist, die Daten aus dem ersten Identifizierungselement (12) auszulesen und/oder die Daten in das erste Identifizierungselement (12) zu schreiben.

4. Vorrichtung nach einem der vorhergegangenen Ansprüche, welche **dadurch gekennzeichnet ist, dass** der Aufbewahrungskörper (13) mindestens einen Sensor (20) umfasst, welcher eingerichtet ist, physische Parameter zum Überwachen des Erhaltungsstatus der medizinischen Substanz in dem Behälter (11) zu ermitteln.

5. Sicherheitsvorrichtung nach Anspruch 4, welche **dadurch gekennzeichnet ist, dass** der Aufbewahrungskörper (13) einen Speicher (21) umfasst, welcher eingerichtet ist, die physischen Parameter, welche von dem Sensor (20) ermittelt werden, zu erhalten und zu speichern.

6. Sicherheitsvorrichtung nach einem der vorhergegangenen Ansprüche, welche **dadurch gekennzeichnet ist, dass** sie zwei erste Identifizierungselemente (12) umfasst, von denen eines der ersten Identifizierungselemente (12) dem Behälter (11) und das andere dem Aufbewahrungskörper (13) zugeordnet ist.

7. Sicherheitsverfahren zum Verfolgen, Steuern und Überwachen der Verabreichung von medizinischen Substanzen, welches umfasst:
- Einbringen einer medizinischen Substanz in einen Behälter (11);
- Einbringen des Behälters (11) in eine Kammer (14) eines Aufbewahrungskörpers (13),
wobei das Verfahren **dadurch gekennzeichnet ist, dass** es umfasst:
- direktes Zuordnen eines ersten Identifizierungselements (12) zu dem Behälter (11) der medizinischen Substanz;
- Schließen der Kammer (14) mit einem Deckel (15);
- Aktivieren eines Schlosses (16), welches der Kammer (14) und dem Deckel (15) zugeordnet ist, um das Öffnen des Deckels (15) zu verhindern;
- Zuordnen eines zweiten Identifizierungselements (18) zu einem tragbaren Element (17), welches einem Patienten zugeordnet werden kann;
- Auslesen und Vergleichen von Daten, welche in dem ersten Identifizierungselement (12) und dem zweiten Identifizierungselement (18) enthalten sind, mit einer Auslesevorrichtung (19), welche fest dem Aufbewahrungskörper (13) zugeordnet ist und mit dem Schloss (16) verbunden ist;
- Senden eines Befehls, um das Schloss (16) zu öffnen, mit der Auslesevorrichtung (19) in Abhängigkeit von dem Vergleichsergebnis.

8. Verfahren nach Anspruch 7, welches **dadurch gekennzeichnet ist, dass** mindestens eines aus dem ersten Identifizierungselement (12) oder dem zweiten Identifizierungselement (18) einen RFID umfasst, welcher eingerichtet ist, die Daten zu speichern, und dadurch, dass das Verfahren mindestens ein Speichern von Daten in dem ersten Identifizierungselement (12) und/oder in dem zweiten Identifizierungselement (18) umfasst.

9. Verfahren nach Anspruch 7 oder 8, welches **dadurch gekennzeichnet ist, dass** es ein Ermitteln und Steuern von physischen Parametern des Behälters (11) mit mindestens einem Sensor (20) umfasst, welche von einem Speicher (21) aufgezeichnet werden.

10. Verfahren nach Anspruch 9, welches **dadurch gekennzeichnet ist, dass** es ein Speichern der physischen Parameter in dem Speicher (21) und ein Analysieren der gespeicherten physischen Parameter mit Schwellenwerten umfasst.

## Revendications

1. Appareil de sécurité pour le suivi, la commande et la surveillance de l'administration de substances médicales, comprenant :
- un récipient (11) de substance médicale ;
- un corps (13) de logement avec un compartiment (14) dans lequel ledit récipient (11) peut être placé, un couvercle (15) pour fermer sélectivement ledit compartiment (14), une serrure (16) associée audit corps (13) de logement et audit couvercle (15) et apte à être sélectivement activée/désactivée pour empêcher ou permettre l'ouverture dudit couvercle (15) ;
**caractérisé en ce qu'**il comprend :
- un premier élément d'identification (12) associé directement audit récipient (11) de la substance médicale ;
- un élément portable (17) pouvant être associé à un patient et pourvu d'un deuxième élément d'identification (18) ;
- un dispositif de lecture (19) configuré pour lire les données dudit premier élément d'identification (12) et dudit deuxième élément d'identification (18), pour comparer lesdites données, et pour commander l'ouverture sélective de ladite serrure (16), ledit dispositif de lecture (19) étant solidement associé audit corps (13) de logement et étant connecté à ladite serrure (16).

2. Appareil selon la revendication 1, **caractérisé en ce qu'**au moins un parmi ledit premier élément d'identification (12) ou ledit deuxième élément d'identification (18) comprend un dispositif d'identification par radiofréquence (RFID) configuré pour mémoriser lesdites données.

3. Appareil selon la revendication 1 ou la revendication 2, **caractérisé en ce qu'**il comprend un dispositif de lecture/écriture (28) configuré pour lire et/ou écrire lesdites données à partir du premier élément d'identification (12) ou respectivement dans celui-ci.

4. Appareil de sécurité selon l'une quelconque des revendications précédentes, **caractérisé en ce que** ledit corps (13) de logement comprend au moins un capteur (20) configuré pour détecter des paramètres physiques afin de surveiller l'état de conservation de ladite substance médicale dans ledit conteneur (11).

5. Appareil de sécurité selon la revendication 4, **caractérisé en ce que** ledit corps (13) de logement comprend une mémoire (21) configurée pour recevoir et mémoriser les paramètres physiques détectés par ledit capteur (20).

6. Appareil de sécurité selon l'une quelconque des revendications précédentes, **caractérisé en ce qu'**il comprend deux premiers éléments d'identification (12), dont l'un des premiers éléments d'identification (12) est associé au conteneur (11) et l'autre est associé au corps (13) de logement.

7. Procédé de sécurité pour le suivi, la commande et la surveillance de l'administration de substances médicales, comprenant :
- le fait d'introduire une substance médicale dans un récipient (11) ;
- le fait d'introduire ledit conteneur (11) dans un compartiment (14) d'un corps (13) de logement ;
**caractérisé en ce qu'**il comprend :
- le fait d'associer un premier élément d'identification (12) directement audit conteneur (11) de la substance médicale ;
- le fait de fermer ledit compartiment (14) avec un couvercle (15) ;
- le fait d'activer une serrure (16), associée audit compartiment (14) et audit couvercle (15) pour empêcher l'ouverture dudit couvercle (15) ;
- le fait d'associer un deuxième élément d'identification (18) à un élément portable (17) apte à être associé à un patient ;
- le fait de lire et de comparer les données contenues dans ledit premier élément d'identification (12) et ledit deuxième élément d'identification (18) avec un dispositif de lecture (19) solidement associé audit corps (13) de logement et étant connecté à ladite serrure (16) ;
- le fait d'envoyer une commande d'ouverture de ladite serrure (16) avec ledit dispositif de lecture (19) en fonction de ladite comparaison.

8. Procédé selon la revendication 7, **caractérisé en ce qu'**au moins un parmi ledit premier élément d'identification (12) ou ledit deuxième élément d'identification (18) comprend un RFID configuré pour mémoriser lesdites données, et **en ce qu'**il comprend au moins une mémorisation de données dans ledit premier élément d'identification (12) et/ou ledit deuxième élément d'identification (18).

9. Procédé selon la revendication 7 ou la revendication 8, **caractérisé en ce qu'**il comprend le fait de détecter et de commander des paramètres physiques dudit conteneur (11) au moyen d'au moins un capteur (20), et enregistrés par une mémoire (21).

10. Procédé selon la revendication 9, **caractérisé en ce qu'**il comprend le fait de mémoriser lesdits paramètres physiques dans ladite mémoire (21), et d'analyser lesdits paramètres physiques mémorisés avec des valeurs de seuil.
